# EUROPEAN PATENT APPLICATION

(11) **EP 4 461 809 A1**
(43) Date of publication of application: **13.11.2024**
(21) Application number: 23735165.5
(22) Date of filing: 03.01.2023
(51) Int. Cl.: C12N 5/0783

(54) **METHOD FOR EXPANSION CULTURE OF GAMMA-DELTA T CELL**

(30) Priority: 03.01.2022 KR 20220000390
(71) Applicant: ImmunoMax Co., Ltd., Seoul 02841 (KR)
(72) Inventor: YOON, Jeongho, Seoul 06716 (KR); NAM, Jae Kook, Seoul 01047 (KR); KIM, Junghoon, Seoul 01882 (KR); RYU, Mihyeun, Seoul 02852 (KR); KIM, Da Jeong, Seoul 02482 (KR); LEE, Minhee, Seoul 02055 (KR); HYUN, Seung-Joo, Seoul 06043 (KR); RYU, Han-Wook, Seoul 02412 (KR); PARK, Mi Sun, Seoul 07667 (KR); LEE, Yeonteak, Seoul 02871 (KR); JUNG, Nahyeon, Seoul 02562 (KR); CHO, Hyo Je, Seoul 02586 (KR)
(74) Representative: Mewburn Ellis LLP
(86) International application number: PCT/KR2023/000082
(87) International publication number: WO 2023/128733

(57) **Abstract**

The present invention relates to a method for producing γδ T cells. According to the present invention, γδ T cells with high cell killing ability and cell viability can be produced in a short period of time with high purity and high efficiency by a clinically friendly method, compared to conventionally known general γδ T cell culture methods or culturing methods using support cells, and thus, there is the advantage of increasing the productivity of allogeneic γδ T cell immunotherapeutic agents.

## Description

### Technical Field

The present invention relates to a method of producing γδ T cells, and more particularly to a method of producing γδ T cells comprising expansion culture of γδ T cells based on inhibition of androgen signaling.

### Background Art

T cells, a type of lymphocyte, are the mainstay of adaptive immunity along with B cells. T cells are created by a process in which precursors made from hematopoietic cells mature in the thymus. T cells are composed of a small number of γδ T cells expressing γδ T cell receptors and the majority of αβ T cells expressing αβ T cell receptors (TCRs) circulating in the thymus and peripheral lymphoid tissue (Non-Patent Document 1). γδ T cells (tissue-resident γδ T cells) resident in epithelial tissues show quite different patterns, and most or all of certain areas may be occupied by γδ T cells (Non-Patent Document 2). γδ T cells resident in epithelial tissues show limited or constant T cell receptor expression patterns, which are different features from T cells resident in lymphoid tissues.

γδ T cells are T cells that are produced in the thymus and migrate to other areas first, and most migrate to the skin, intestines, lungs, and epithelial tissues before birth (Non-Patent Document 3). Although γδ T cells do not have the diversity of T cell receptors, they recognize and respond to various biological damage inflicted on epithelial tissues, but little is known about autoantigens that induce these responses. Activated γδ T cells are known to control inflammation, destroy cells with changed cell characteristics, and improve wound healing (Non-Patent Document 3). In addition, according to recent research results, γδ T cells are known to act as regulators that maintain and restore homeostasis in tissues exposed to stimuli from the external environment (Non-Patent Document 1) .

γδ T cells are present in a very small proportion of about 0.5 to 5% in human peripheral blood, and are known to be functionally involved in both immune responses, adaptive immunity and innate immunity. γδ T cells, which play an important role in the immune response, are known to respond to phosphoantigens produced by cancer cells or bacteria.

Human γδ T cells have two main subsets of TCR chains: Vδ1 or Vδ2 chains (Non-Patent Document 4), and most γδ T cells in the blood have Vδ2 chains paired with Vγ9 chains. TCR γδ T cells develop before TCR αβ T cells in the thymus, and have limited TCR diversity against several antigens, including phosphoantigens, but initiate an immune response faster than TCR αβ T cells, inducing an innate-like immune response. γδ T cells, like αβ T cells, have a more potent anticancer effect through cytolysis, but are known to have no MHC restriction and cause no graft-versus-host disease, unlike αβ T cells that are the main cause of graft-versus-host disease (GVHD) on cells expressing non-self MHCs (Non-Patent Document 5).

Therefore, γδ T cells are a promising candidate cell group for anticancer immune cell therapy, and since the discovery that γδ T cells specifically proliferate by treatment with interleukin-2 (IL-2) and zoledronic acid (ZA, ZOL), the therapeutic effect in various types of cancer has been proven by preclinical and clinical trials. The development of cancer immunotherapy, including haploidentical transplantation using γδ T cells and amplification of γδ T cells with zoledronic acid and interleukin-2, is expected to maximize treatment efficacy for high-risk patients with refractory disease.

Most γδ T cells in the body are present in a very small proportion of about 0.5 to 5% in the peripheral blood, and in order to actually use γδ T cells for therapeutic purposes, a large amount of activated γδ T cells is required. Hence, methods in which γδ T cells isolated from the blood are mass cultured *in vitro* are being actively studied. For culturing γδ T cells, methods using not only zoledronic acid and IL-2, which have been conventionally used for cell proliferation/activation, but also IL-15 (Patent Documents 1 and 2), methods using PHA (phytohemagglutinin), TGF-β, concanavalin A, amphotericin B, etc. (Patent Documents 3 to 6), and the like have been developed, but this is a modification and development in the use of IL-2, which has been used conventionally, to find a new proliferation material, and does not suggest innovative or improved methods. Also, cases of amplifying γδ T cells using feeder cells have been reported. When using feeder cells, specific cancer cell lines, rather than normal cells, are transformed or transduced and used for mass proliferation of γδ T cells (Non-Patent Document 6).

This method is not suitable for ensuring safety, which is important for clinical applications, and has the limitation of producing γδ T cells with priming specificity for specific cancer cells.

Accordingly, the present inventors have made great efforts to develop a method of obtaining TCR γδ T cells by mass expansion culture, and thus ascertained that, when undifferentiated T cells isolated from human peripheral blood mononuclear cells are cultured in media comprising finasteride, which is a 5α-reductase inhibitor, as an active ingredient, in addition to conventionally known stimulants zoledronic acid and IL-2, growth of γδ T cells may be increased and cell yield may be improved, thus culminating in the present invention.

### Prior art

### Patent Literature

(Patent Document 1) 1. U.S. Patent No. 11,135,245 B
(Patent Document 2) 2. European Patent No. 3,154,567 B
(Patent Document 3) 3. U.S. Patent No. 10,370,452 B
(Patent Document 4) 4. U.S. Patent No. 10,557,117 B2
(Patent Document 5) 5. WO 2020-172555
(Patent Document 6) 6. U.S. Patent Application Publication No. 2019-0175650 A

### Non-Patent Literature

(Non-Patent Document 1) Jameson J., Havan W.L., Immunol. Rev., 215:114-22., 2007
(Non-Patent Document 2) Allison J.P., Havran W.L., Annu. Rev. Immunol., 9:679-705, 1991
(Non-Patent Document 3) Komori H.K., Meehan T.F., Havran W.L., Curr. Opin. Immunol., 18(5):534-8, 2006
(Non-Patent Document 4) Kabelitz D., Marischen, L., Oberg, H.-H., Holtmeier, W.; Wesch, D. Int. Arch. Allergy Immunol., 137, 73-81, 2005
(Non-Patent Document 5) Sharma A., Zumwalde N.A., Gumperz, J., In Advanced Structural Safety Studies; Humana Press Inc.: New York, NY, USA, pp. 57-72, 2019
(Non-Patent Document 6) Tan W.K., Tay J.C.K., Zheng J., Zheng M., Wang S., J. Immunol Sci., 2(3): 6-12, 2018

### Summary of the Invention

It is an object of the present invention to provide a method of producing γδ T cells capable of increasing the growth of γδ T cells and improving cell yield.

It is another object of the present invention to provide a medium composition for inducing and culturing γδ T cells capable of improving the growth of γδ T cells and cell yield.

In order to accomplish the above objects, the present invention provides a method of producing γδ T cells, comprising:
(a) inducing and culturing γδ T cells by culturing peripheral blood mononuclear cells (PBMCs) in medium comprising (i) zoledronic acid, (ii) IL-2, and (iii) a 5α-reductase inhibitor or an androgen receptor antagonist; and
(b) obtaining the cultured γδ T cells.

The present invention also provides a medium composition for inducing and culturing γδ T cells comprising (i) zoledronic acid, (ii) IL-2, and (iii) a 5α-reductase inhibitor or an androgen receptor antagonist.

### Brief Description of Drawings

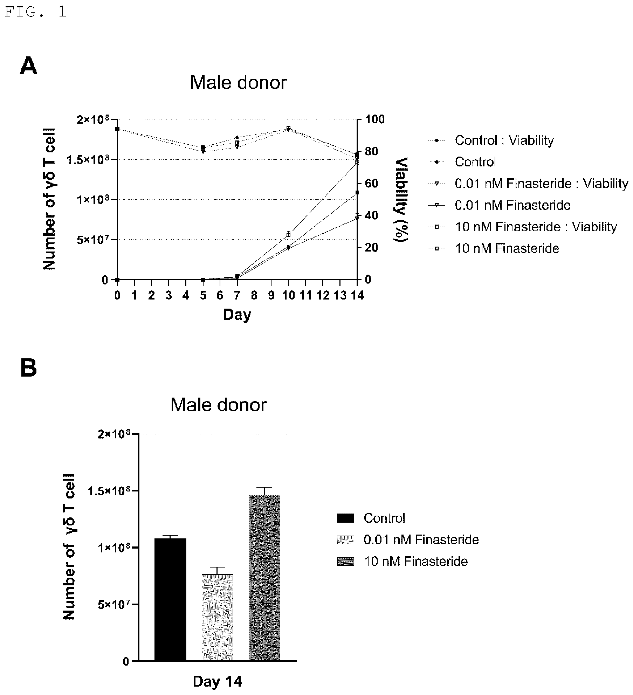

FIG. 1 shows results of a comparative analysis of the proliferation rate and viability of γδ T cells for a 14-day culture period by continuous treatment with basic culture components and finasteride at different concentrations during culture of γδ T cells using peripheral blood mononuclear cells derived from male donors.

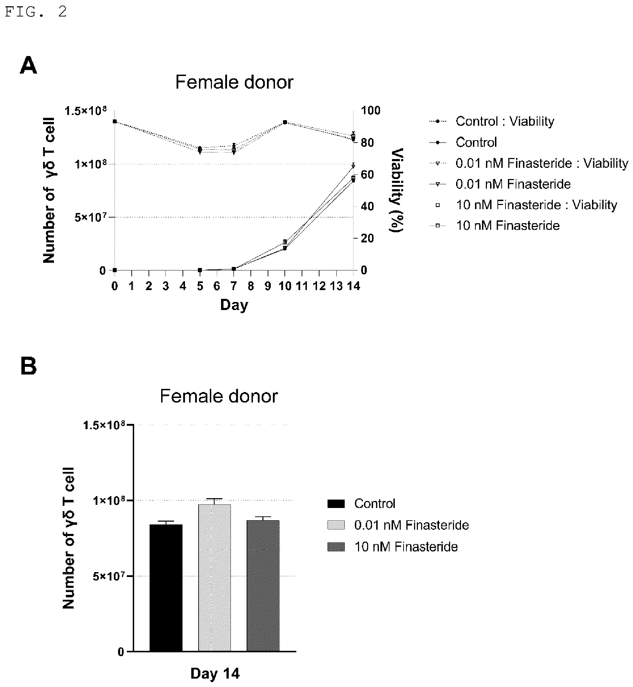

FIG. 2 shows results of a comparative analysis of the proliferation rate and viability of γδ T cells for a 14-day culture period by continuous treatment with the basic culture components and finasteride at different concentrations during culture of γδ T cells using peripheral blood mononuclear cells derived from female donors.

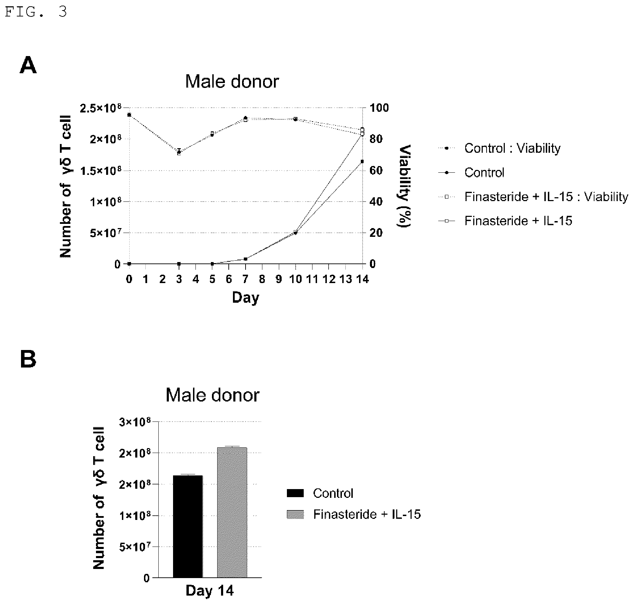

FIG. 3 shows results confirming the proliferation and viability of γδ T cells depending on treatment with finasteride and IL-15 using peripheral blood mononuclear cells derived from male donors, which are results of a comparative analysis of γδ T cell proliferation and viability of male donors by continuous treatment with a high concentration (10 nM) of finasteride until day 7 and with IL-15 for a 14-day culture period (A), and the increased number of γδ T cells compared to the control group after 14 days of culture (B).

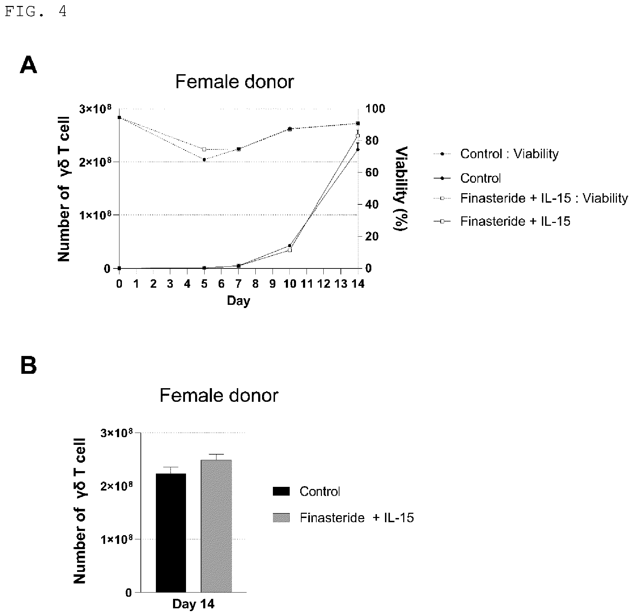

FIG. 4 shows results confirming the proliferation and viability of γδ T cells depending on treatment with finasteride and IL-15 using peripheral blood mononuclear cells derived from female donors, which are results of a comparative analysis of γδ T cell proliferation and viability of female donors by continuous treatment with a low concentration (0.01 nM) of finasteride until day 7 and with IL-15 for a 14-day culture period (A), and the increased number of γδ T cells compared to the control group after 14 days of culture (B).

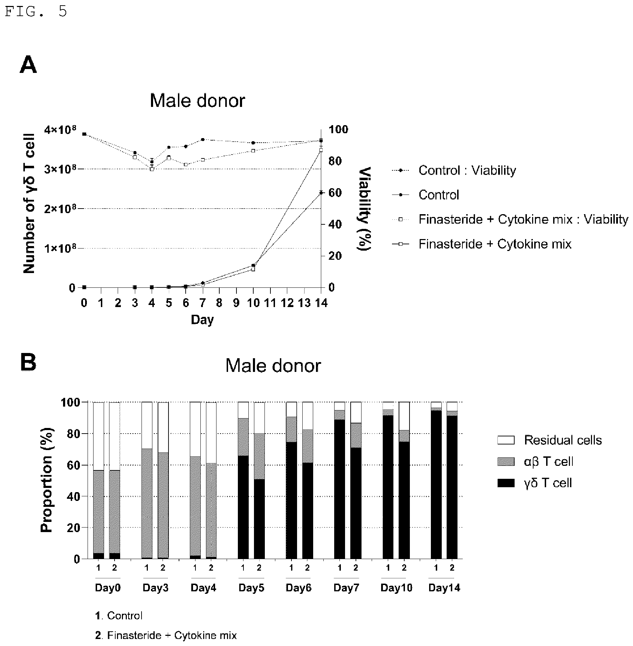

FIG. 5 shows results confirming the proliferation and viability of γδ T cells depending on treatment with finasteride (10 nM) and cytokine mix using peripheral blood mononuclear cells derived from male donors, in which A shows results of a comparison of the proliferation rate and viability of γδ T cells for 14 days, and B shows results of analyzing the proportions of cultured γδ T cells and αβ T cells.

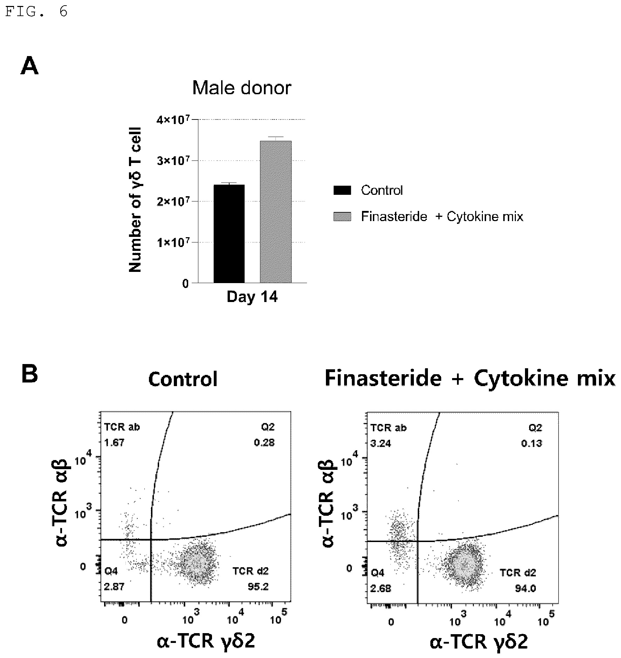

FIG. 6 shows results confirming the proliferation and viability of γδ T cells depending on treatment with finasteride (10 nM) and cytokine mix using peripheral blood mononuclear cells derived from male donors, in which A shows results of a comparative analysis of the number of γδ T cells after 14 days of culture, and B shows results of analyzing the proportions of γδ T cells compared to the control group using flow cytometry after 14 days of culture.

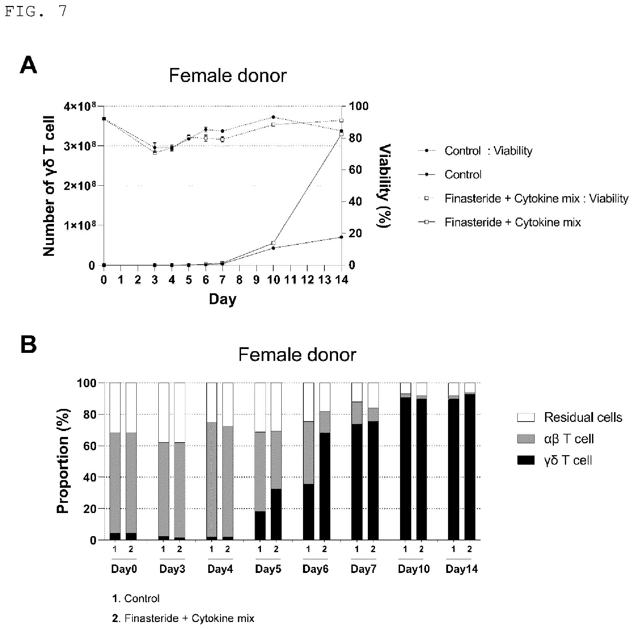

FIG. 7 shows results confirming the proliferation and viability of γδ T cells depending on treatment with finasteride (0.01 nM) and cytokine mix using peripheral blood mononuclear cells derived from female donors, in which A shows results of a comparison of the proliferation rate and viability of γδ T cells for 14 days, and B shows results of analyzing the proportions of cultured γδ T cells and αβ T cells.

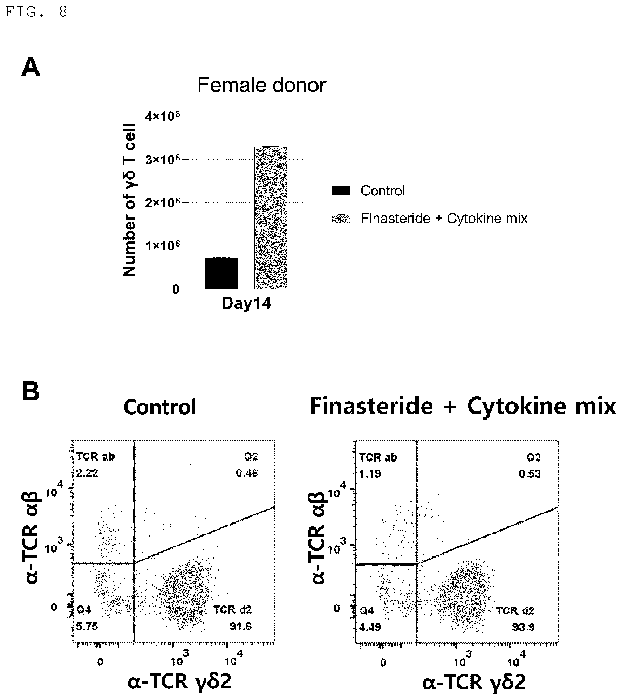

FIG. 8 shows results confirming the proliferation and viability of γδ T cells depending on treatment with finasteride (0.01 nM) and cytokine mix using peripheral blood mononuclear cells derived from female donors, in which A shows results of a comparative analysis of the number of γδ T cells after 14 days of culture, and B shows results of analyzing the proportions of γδ T cells compared to the control group using flow cytometry after 14 days of culture.

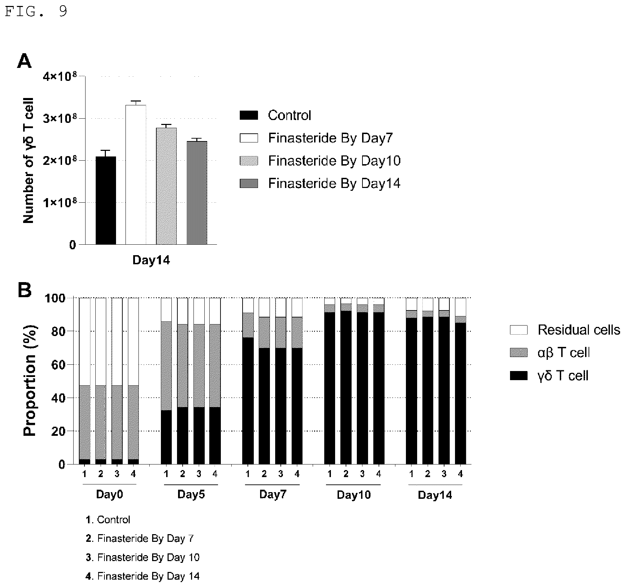

FIG. 9 A shows results of a comparative analysis of the number of γδ T cells on day 14 by continuous treatment with finasteride for 7, 10, and 14 days from the start date of culture using peripheral blood mononuclear cells from healthy donors, and B shows results of analyzing the proportions of γδ T cells and αβ T cells cultured until day 14 using the above culture process.

### Detailed Description and Preferred Embodiments of the Invention

Unless otherwise defined, all technical and scientific terms used herein have the same meanings as typically understood by those skilled in the art to which the present invention belongs. In general, the nomenclature used herein is well known in the art and is typical.

In the present invention, attempts have been made to develop a method for explosively proliferating γδ T cells from peripheral blood mononuclear cells in a short period of time, confirming that, when culturing peripheral blood mononuclear cells in serum medium comprising a 5α-reductase inhibitor or an androgen receptor antagonist as an androgen inhibitor, the proliferation rate and viability of γδ T cells may be increased.

Accordingly, an aspect of the present invention relates to a method of producing γδ T cells, comprising:
(a) inducing and culturing γδ T cells by culturing peripheral blood mononuclear cells (PBMCs) in medium comprising (i) zoledronic acid, (ii) IL-2, and (iii) a 5α-reductase inhibitor or an androgen receptor antagonist; and
(b) obtaining the cultured γδ T cells.

Zoledronic acid used in the present invention is a bisphosphonate-based drug, and is used to treat hypercalcemia caused by cancer such as Paget's disease, metastatic breast cancer, parathyroid gland tumors, multiple myeloma, etc. Zoledronic acid has a structure similar to endogenous pyrophosphate that regulates calcium concentration in the blood, and unlike existing bisphosphonates (clodronate, etidronate, tiludronate, etc.), zoledronic acid has a nitrogen-containing imidazole ring, exhibiting a very strong effect of inhibiting bone resorption. Zoledronic acid inhibits the reabsorption of calcium into the blood by chelating calcium in hydroxyapatite, which is a bone matrix component. Also, by inhibiting farnesyl diphosphate synthase, prenylation of guanine nucleotide-binding protein (G-protein) in osteoclasts is disrupted to induce apoptosis of osteoclasts that promote bone resorption, thus reducing osteoclasts, thereby controlling the calcium concentration in the blood. Zoledronic acid is quickly absorbed from the blood into the bone and stored in the bone, and the half-life thereof in bone is long, so bone resorption may be sustained by a single intravenous injection. However, bisphosphonates in the blood have a very short half-life of 0.5-2 hours, and are excreted through the kidneys without being metabolized, so dosage adjustment is necessary for patients with kidney damage.

In the present invention, 5α-reductase is a biocatalyst that reduces the male hormone testosterone into dihydrotestosterone (DHT), and is present as type 1 to type 3. When type 1 abnormalities occur, bone weight and muscle mass decrease, and when type 2 abnormalities occur, typical intersex symptoms occur. When type 3 abnormalities occur, cell growth and vision (eye) abnormalities are caused, and production of testosterone, androstenedione, and progesterone is decreased.

It is known that 5α-reductase inhibitors decrease the amount of activated substrate by inhibiting the conversion of the substrate into activated form, resulting in a slight increase in the level of the substrate. The mechanism of inhibition of 5α-reductase involves binding of NADPH to the enzyme followed by the substrate. Specific substrates include testosterone, progesterone, androstenedione, epitestosterone, cortisol, aldosterone, deoxycorticosterone, etc.

Substrate + NADPH + H+ → 5α-Substrate + NADP+

5α-reductase isoforms Type I and II reduce testosterone into dihydrotestosterone (DHT), progesterone into 5α-dihydroprogesterone (5α-DHP), and deoxycorticosterone into dihydrodeoxycorticosterone (DHDOC) .

Finasteride used in the present invention is a 5α-reductase inhibitor that inhibits the conversion of testosterone into activated form, dihydrotestosterone. Dihydrotestosterone, which is the activated form of testosterone, is known to be involved in hair loss and benign prostatic hyperplasia. Therefore, a 5α-reductase inhibitor, which inhibits the production of dihydrotestosterone in tissues, is used as a therapeutic agent for benign prostatic hyperplasia and is also used to prevent and treat hair loss.

In the present invention, the 5α-reductase inhibitor may be finasteride, dutasteride, epristeride, alfatradiol, Saw Palmetto extract, etc., but is not limited thereto.

In the present invention, the androgen receptor antagonist may be bicalutamide, flutamide, nilutamide, etc., but is not limited thereto.

The 5α-reductase inhibitor used in the present invention is preferably finasteride.

In the present invention, the medium may additionally comprise a costimulatory molecule, and in the present invention, the costimulatory molecule that may be comprised in the culture medium is a peripheral cell membrane protein that appears in activated antigen-presenting cells (APCs), and is a general term for cell surface membrane proteins that may interact with the surface membrane proteins of T cells to create costimulatory signals that increase or decrease MHC-TCR signaling between APCs and T cells. As the costimulatory molecule, an antibody or a ligand may be used, and examples thereof may comprise antibodies or ligands to 5A6.E9, Bl, TS8.2, 15D, B6, B3, TS-1, γ3.20, 7A5, IMMU510, R9.12, 11F2 or combinations thereof, phorbol 12-myristate 13-acetate (TPA), mezerein, Staphylococcus enterotoxin A (SEA), Streptococcus protein A, amphotericin B or combinations thereof, αTCR, βTCR, γTCR, δTCR, CD277, CD28, CD46, CTLA4, ICOS, PD-1, CD30, NKG2D, NKG2A, HVEM, 4-1BB (CD137), OX40 (CD134), CD70, CD80, CD86, DAP, CD122, GITR, FceRIg, CD1, CD16, CD161, DNAX, accessory molecule-1 (DNAM-1), SLAM, Coxsackievirus and adenovirus receptors or combinations thereof. Preferably, CD80, 4-1BB, CD86, or CD276 is used, and the costimulatory molecule is not limited to CD80 used in examples of the present invention, and it is obvious to those skilled in the art that other costimulatory molecules may be used without limitation so long as they meet the purpose of the present invention.

The concentration of the costimulatory molecule used for culturing γδ T cells of the present invention in the medium is 1 to 2,000 nM, particularly 5 to 1,000 nM, more particularly 10 to 500 nM, and the concentration of the cytokine in the medium is 1 to 2,000 IU, particularly 10 to 1,000 IU, more particularly about 20 to 400 IU.

In the present invention, the cytokine that may be comprised in the culture medium may be at least one selected from among interleukins. Interleukin is a general term for protein-based bioactive materials produced by immune cells, and the interleukin that may be used in the present invention may be at least one selected from the group consisting of interleukin-2 (IL-2), interleukin-12 (IL-12), interleukin-15 (IL-15), and interleukin-18 (IL-18), and the interleukin used in the present invention may comprise, but is not limited to, IL-2, IL-15, and IL-18, and it is obvious to those skilled in the art that other cytokines may be used without limitation so long as they meet the purpose of the present invention.

In the present invention, in mass expansion culture of TCR γδ T cells, finasteride, an androgen signaling inhibitor, is used, and simultaneously, treatment with zoledronic acid and IL-2 is performed, and in the above method, when IL-15 and IL-18 cytokines and the CD80 costimulatory molecule are additionally used, mass proliferation of TCR γδ T cells is possible with higher efficiency. As such, a cell concentration of 0.4×10⁶ to 3.0×10⁶ cells/ml has to be maintained for the total culture period. Stationary culture of γδ T cells while maintaining the above conditions enables mass expansion culture of γδ T cells.

In an aspect of the present invention, the method of culturing γδ T cells is as follows:
(a) stimulating a culture fluid of peripheral blood mononuclear cells comprising γδ T cells, followed by stationary culture;
(b) diluting the cell culture fluid subjected to stationary culture to a predetermined cell proportion after a predetermined period of time, followed by stationary culture; and
(c) performing stationary culture by adding a culture medium comprising an additional ingredient after re-inducing the culture fluid comprising the cells subjected to stationary culture when the number of γδ T cells in the cell culture fluid reaches a predetermined level.

After step (c), collecting mass produced γδ T cells may be further comprised.

The reaction vessel that may be used for stationary culture in the present invention may comprise, but is not limited to, a 24-well plate, 12-well plate, 6-well plate, shaking flask, T25-flask (T-flask), T75-flask (T-flask), T175-flask (T-flask), T225-flask (T-flask), disposable cell culture bag, etc., and any reaction vessel that may be easily adopted by those skilled in the art may be used.

In the present invention, the term "appropriate culture conditions" may refer to stationary culture of cells in a 5% CO₂ incubator at 37°C.

In the present invention, the term "predetermined culture period" may refer to a period of about 2 to 5 days during which γδ T cells proliferate and reach a predetermined proportion or a predetermined number of cells.

In the present invention, the term "predetermined proportion" or "predetermined number of cells" may refer to the number of cells in which γδ T cells proliferate for a predetermined culture period and have a concentration of 0.4×10⁶ to 3.0×10⁶ cells/ml within a predetermined culture period.

In the present invention, the term "re-induction" may refer to re-inducing γδ T cell proliferation by resuspending the aggregated cells in a culture fluid comprising cells using a micropipette or a serological pipette after a predetermined culture period and further adding a cell culture medium and a cytokine mix.

In the present invention, the term "cytokine mix" may refer to a mixed additive of IL-15 and IL-18 cytokines, a CD80 costimulatory factor, and an additional active ingredient, which is further added after re-induction for the culture period to induce re-induction and cell proliferation.

In the present invention, it is preferable to use finasteride, which is a 5α-reductase inhibitor, as the active ingredient, but the present invention is not limited thereto, and the active ingredient may also comprise dutasteride, epristeride, alfatradiol, Saw Palmetto extract, etc. and an androgen receptor antagonist (ARA) such as bicalutamide, flutamide, nilutamide etc.

The basic culture medium used in the present invention is Gibco CTS OpTmizer T cell Expansion Serum Free Media (Thermo-Fisher Scientific, Cat# A1048501), but is not limited thereto, and typical T cell culture medium for culturing immune cells comprising T cells, for example, Gibco CTS AIM-V medium (Thermo-Fisher Scientific), LymphoONE T cell Expansion XFM (Takara), X-VIVO 10, -15, & -20 Hematopoietic Cell Medium (Lonza), PRIME-SV T cell Expansion XSFM (Fujifilm-Irvine Scientific), and RPMI-1640 medium, may be used.

Finasteride used in the present invention may be comprised at a concentration of 0.001 to 100 nM, preferably 0.001 to 50 nM in the γδ T cell induction and culture medium, and the optimal concentration of finasteride that is added may vary depending on whether the peripheral blood mononuclear cells used for γδ T cell induction and culture are derived from males or females.

When the peripheral blood mononuclear cells used for γδ T cell induction and culture in the present invention are derived from males, the γδ T cell induction and culture medium preferably comprises 1 to 50 nM finasteride, more preferably 1 to 20 nM finasteride.

When the peripheral blood mononuclear cells used for γδ T cell induction and culture in the present invention are derived from females, the γδ T cell induction and culture medium preferably comprises 0.001 to 10 nM finasteride, more preferably 0.001 to 1 nM finasteride.

In the present invention, culturing γδ T is preferably performed for a total of 3 to 21 days, more preferably for 7 to 14 days.

According to the method of inducing and culturing γδ T of the present invention, cells in which the proportion of γδ T cells in the total cultured cells on day 14 of culture is 90% or more may be obtained.

Another aspect of the present invention relates to a medium composition for inducing and culturing γδ T cells comprising (i) zoledronic acid, (ii) IL-2, and (iii) a 5α-reductase inhibitor or an androgen receptor antagonist.

In the present invention, the 5α-reductase inhibitor may be, but is not limited to, finasteride, dutasteride, epristeride, alfatradiol, Saw Palmetto extract, etc.

In the present invention, the androgen receptor antagonist may comprise, but is not limited to, bicalutamide, flutamide, nilutamide, etc.

The 5α-reductase inhibitor used in the present invention is preferably finasteride.

In the present invention, the medium may further comprise a costimulatory molecule, and in the present invention, the costimulatory molecule that may be comprised in the culture medium is a peripheral cell membrane protein that appears in activated antigen-presenting cells (APCs), and is a general term for cell surface membrane proteins that may interact with the surface membrane proteins of T cells to create costimulatory signals that increase or decrease MHC-TCR signaling between APCs and T cells. The costimulatory molecule may comprise an antibody or a ligand, examples of which may comprise antibodies or ligands to 5A6.E9, Bl, TS8.2, 15D, B6, B3, TS-1, γ3.20, 7A5, IMMU510, R9.12, 11F2 or combinations thereof, phorbol 12-myristate 13-acetate (TPA), mezerein, Staphylococcus enterotoxin A (SEA), Streptococcus protein A, amphotericin B or combinations thereof, αTCR, βTCR, γTCR, δTCR, CD277, CD28, CD46, CTLA4, ICOS, PD-1, CD30, NKG2D, NKG2A, HVEM, 4-1BB (CD137), OX40 (CD134), CD70, CD80, CD86, DAP, CD122, GITR, FceRIg, CD1, CD16, CD161, DNAX, accessory molecule-1 (DNAM-1), SLAM, Coxsackievirus and adenovirus receptors or combinations thereof. Preferably, CD80, 4-1BB, CD86, or CD276 is used, and the costimulatory molecule is not limited to CD80 used in examples of the present invention, and it is obvious to those skilled in the art that other costimulatory molecules may be used without limitation so long as they meet the purpose of the present invention.

The concentration of the costimulatory molecule used for culturing γδ T cells of the present invention in the medium is 1 to 2,000 nM, particularly 5 to 1,000 nM, more particularly 10 to 500 nM, and the concentration of the cytokine in the medium is 1 to 2,000 IU, particularly 10 to 1,000 IU, more particularly about 20 to 400 IU.

In the present invention, the cytokine that may be comprised in the culture medium may be at least one selected from among interleukins. Interleukin is a general term for protein-based bioactive materials produced by immune cells, and the interleukin that may be used in the present invention may be at least one selected from the group consisting of interleukin-2 (IL-2), interleukin-12 (IL-12), interleukin-15 (IL-15), and interleukin-18 (IL-18), and the interleukin used in the present invention may comprise, but is not limited to, IL-2, IL-15, and IL-18, and it is obvious to those skilled in the art that other cytokines may be used without limitation so long as they meet the purpose of the present invention.

A better understanding of the present invention may be obtained through the following examples. These examples are merely set forth to illustrate the present invention, and are not to be construed as limiting the scope of the present invention, as will be apparent to those of ordinary skill in the art.

### Example 1. Confirmation of proliferation rate and viability of γδ T cells in healthy peripheral blood depending on finasteride concentration

### 1-1. Isolation of human peripheral blood mononuclear cells (hPBMCs)

For 100 cc of blood from a healthy donor collected by a medical professional and placed in a heparin tube, 25 ml of 1X DPBS and 25 ml of the blood were placed in four 50 ml conical tubes. 15 ml of Lymphoprep (Axis Shield, UK) was vertically dispensed into a new 50 ml conical tube carefully to avoid contacting the wall as much as possible, and the diluted blood was slowly added to the top of the 50 ml conical tube containing the Lymphoprep solution along the wall. The conical tube was placed in a centrifuge and centrifuged at 800 × g (1,890 rpm) for 20 minutes at room temperature. In the separated four blood layers, the white buffy coat layer between Lymphoprep and plasma layers was collected, transferred to new 50 ml conical tubes (three or four), and centrifuged at 1,500 rpm (500 × g) for 10 minutes at room temperature. The cells divided into three or four 50 ml conical tubes were resuspended in 45 ml of 1X DPBS and then collected into one 50 ml conical tube. The cells collected in one tube were centrifuged a total of two times at 1,250 rpm (350 × g) for 10 minutes at room temperature. A culture medium was added to a stock of 1.0×10⁷ cells to a final volume of 10 ml so that the cell concentration was 1.0×10⁶ cells/ml, followed by pipetting two or three times, after which 1.0 ml of the cells was dispensed as day 0 samples to proceed with culture, and the remaining cells were dispensed at 1.0×10⁷ cells into frozen vials, stored frozen in a nitrogen tank (LN₂ tank), and used by thawing as necessary.

### 1-2. Proliferation rate and viability of γδ T cells in peripheral blood mononuclear cells depending on finasteride concentration

The peripheral blood mononuclear cells (PBMC) isolated in 1-1 were continuously treated with 3 µM zoledronic acid and 10³ IU/ml IL-2 as basic culture components for γδ T cells and with 0.01 nM and 10 nM finasteride as an active ingredient, followed by culture for a total of 14 days, and the activation and proliferation rates of γδ T cells were compared.

The frozen peripheral blood mononuclear cells were thawed in a constant temperature water bath at 37°C and then suspended in 9 ml of OpTmizer medium (Thermo-Fisher Scientific, USA) containing 10% heat inactivated fetal bovine serum (FBS, Gibco, USA). The number of cells was measured, followed by centrifugation at 400 × g and 25°C for 4 minutes. The cell pellets obtained by centrifugation were suspended in 5 ml of complete OpTmizer medium (10% fetal bovine serum + OpTmizer medium, cOpTmizer) and the number of cells was measured, after which the cells were diluted with cOpTmizer medium to a concentration of 1.0×10⁶ cells/ml.

A total of 1.×10⁶ cells (final 1 ml) was seeded per well in a 24-well plate. To stimulate and induce differentiation of γδ T cells, 0.01 nM and 10 nM finasteride, 3 µM zoledronic acid, and IL-2 (10³ IU/ml) were added, followed by culture in a CO₂ incubator at 37°C for 3 days.

3 days after the start of culture, the aggregated cells were resuspended using a 1000P micropipette, and the same amount of cOpTmizer culture medium and 0.01 nM and 10 nM finasteride and IL-2 (10³ IU/ml) for re-induction were further added, followed by culture in a CO₂ incubator at 37°C for 2 days.

After 2 days of re-induction, the aggregated cells were resuspended using a 1000P micropipette and the number of cells was measured. The same amount of cOpTmizer culture medium based on the cell culture fluid in culture and 0.01 nM and 10 nM finasteride and IL-2 (10³ IU/ml) for re-induction were further added, followed by culture in a CO₂ incubator at 37°C.

6 days after the start of culture, half of the cOpTmizer culture medium based on the cell culture fluid in culture and 0.01 nM and 10 nM finasteride and IL-2 (10³ IU/ml) for re-induction were further added, followed by culture in a CO₂ incubator at 37°C.

7 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells based on the cells in culture, after which IL-2 (10³ IU/ml) was added to the additional cOpTmizer culture medium, followed by culture in a CO₂ incubator at 37°C for 3 days.

10 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells based on the cells in culture. IL-2 (10³ IU/ml) was added to the additional cOpTmizer culture medium, followed by culture in a CO₂ incubator at 37°C for 2 days.

12 days after the start of the culture, the same amount of cOpTmizer culture medium based on the cell culture fluid medium in culture was added and IL-2 (10³ IU/ml) was further added to the additional cOpTmizer culture medium, followed by culture in a CO₂ incubator at 37°C.

14 days after the start of culture, the aggregated cells were resuspended using a 1000P micropipette or a 10 ml serological pipette and the number of cells was measured. In Example 1, the compositions and proportions of cells including αβ T cells and γδ T cells were analyzed using Fortessa-X20 flow cytometry from BD Bioscience on the start date of culture, day 5, day 7, day 10, and day 14 using the antibodies shown in Table 1 below.

Thereby, as shown in FIGs. 1 and 2, when the peripheral blood mononuclear cells isolated from male and female donors were treated with finasteride at concentrations of 0 nM, 0.01 nM, and 10 nM and cultured for 14 days, for male donors, the proliferation rate of γδ T cells increased at a high concentration of finasteride compared to the control group treated with only IL-2 and ZA (FIG. 1), and for female donors, contrary to male donors, the proliferation rate of γδ T cells increased at a low concentration of finasteride (FIG. 2).

**[Table 1]**

| Antibody information used for T cell phenotype analysis | | | |
|---|---|---|---|
| **Target** | **Fluorescence** | **Manufacturer** | **Catalog number** |
| CD3 | APC | BD Bioscience | 555335 |
| TCR αβ | APC·Cy7 | BioLegend | 306728 |
| TCR Vδ2 | FITC | BD Bioscience | 555738 |

### Example 2. Proliferation rate and viability of γδ T cells in peripheral blood mononuclear cells depending on treatment with finasteride and IL-15

Peripheral blood mononuclear cells (PBMC) were isolated from healthy donors and were cultured for a total of 14 days by continuous treatment with finasteride as the active ingredient at 10 nM for male donors or at 0.01 nM for female donors until day 7 and with 0.8 nM IL-15, in addition to the basic culture components for γδ T cells (3 µM zoledronic acid (ZA) and 10³ IU/ml IL-2), and the activation and proliferation rates of γδ T cells were compared.

The frozen peripheral blood mononuclear cells were thawed in a constant temperature water bath at 37°C and suspended in 9 ml of OpTmizer medium (cOpTmizer, Thermo-Fisher Scientific, USA) containing 10% fetal bovine serum (FBS). The number of cells was measured, followed by centrifugation at 400 × g and 25°C for 4 minutes. The cell pellets obtained by centrifugation were suspended in 5 ml of cOpTmizer medium and the number of cells was measured, after which the cells were diluted to a concentration of 1.0×10⁶ cells/ml.

A total of 1.0×10⁶ cells (final 1 ml) was seeded per well in a 24-well plate. To stimulate and induce differentiation of γδ T cells, finasteride at 10 nM for males and 0.01 nM for females, 3 µM zoledronic acid (ZA), IL-2 (10³ IU/ml), and 0.8 nM IL-15 were added, followed by culture in a CO₂ incubator at 37°C for 3 days.

3 days after the start of culture, the aggregated cells were resuspended using a 1000P micropipette and the number of cells was measured. The same amount of cOpTmizer culture medium, IL-2 (10³ IU/ml), finasteride at 10 nM for males and 0.01 nM for females, and 0.8 nM IL-15 were further added, followed by culture in a CO₂ incubator at 37°C for 2 days.

After 2 days of resuspension, the aggregated cells were resuspended using a 1000P micropipette and the number of cells was measured. The same amount of cOpTmizer culture medium based on the cell culture fluid in culture, IL-2 (10³ IU/ml), finasteride at 10 nM for males and 0.01 nM for females, and 0.8 nM IL-15 were further added, followed by culture in a CO₂ incubator at 37°C.

6 days after the start of culture, finasteride at 10 nM for males and 0.01 nM for females, IL-2 (10³ IU/ml), and 0.8 nM IL-15 were further added to half of the cOpTmizer culture medium based on the cell culture fluid in culture, followed by culture in a CO₂ incubator at 37°C.

7 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells/ml based on the cells in culture, after which IL-2 (10³ IU/ml) and 0.8 nM IL-15 were further added to the additional cOpTmizer culture medium, followed by culture in a CO₂ incubator at 37°C for 3 days.

10 days after the start of culture, the aggregated cells were resuspended using a 1000P micropipette or a 10 ml serological pipette and then the number of cells was measured. The cells were diluted with cOpTmizer culture medium to a concentration of 0.4×10⁶ cells/ml based on the cells in culture. IL-2 (10³ IU/ml) and 0.8 nM IL-15 were further added to the additional cOpTmizer culture medium, followed by culture in a CO₂ incubator at 37°C.

12 days after the start of culture, IL-2 (10³ IU/ml) and 0.8 nM IL-15 were further added to the same amount of cOpTmizer culture medium based on the cell culture fluid in culture, followed by culture in a CO₂ incubator at 37°C.

The next day, IL-2 (10³ IU/ml) and 0.8 nM IL-15 were further added to half of the cOpTmizer culture medium based on the cell culture fluid in culture, followed by culture in a CO₂ incubator at 37°C.

14 days after the start of culture, the aggregated cells were resuspended using a 1000P micropipette and then the number of cells was measured. In Example 2, the compositions and proportions of cells including αβ T cells and γδ T cells were analyzed using Fortessa-X20 flow cytometry from BD Bioscience on the start date of culture, day 5, day 7, day 10, and day 14 using the antibodies shown in Table 1.

Thereby, as shown in FIGs. 3 and 4, for male donors, the proliferation rate of γδ T cells increased 1.26 times (126%) compared to the control group treated with only IL-2 and ZA (FIG. 3), and for female donors, unlike male donors, there was no significant difference in the proliferation rate of γδ T cells (FIG. 4).

### Example 3. Proliferation rate and viability of γδ T cells in healthy peripheral blood depending on treatment with finasteride, IL-15, IL-18, and CD80

Peripheral blood mononuclear cells (PBMC) were isolated from healthy donors and were cultured for a total of 14 days by single treatment with finasteride as the active ingredient at 10 nM for males and 0.01 nM for females on the start date of culture, single treatment with 6 nM IL-18 as the additional ingredient on the start date of culture, and continuous treatment with 100 nM CD80 and 0.8 nM IL-15, in addition to the basic culture components for γδ T cells (3 µM zoledronic acid and 10³ IU/ml IL-2), and the activation and proliferation rates of γδ T cells were compared.

The frozen peripheral blood mononuclear cells were thawed in a constant temperature water bath at 37°C and then suspended in 9 ml of OpTmizer medium (cOpTmizer, Thermo-Fisher Scientific, USA) containing 10% fetal bovine serum (FBS). The number of cells was measured, followed by centrifugation at 400 × g and 25°C for 4 minutes. The cell pellets obtained by centrifugation were suspended in 5 ml of cOpTmizer medium (Thermo-Fisher Scientific, USA) and the number of cells was measured, after which the cells were diluted with cOpTmizer medium to a concentration of 1.0×10⁶ cells/ml.

A total of 1.0×10⁶ cells was dispensed at 1 ml per well in a 24-well plate. To stimulate and induce differentiation of γδ T cells, the cells were treated with finasteride as the active ingredient at 10 nM for male donors and 0.01 nM for female donors, and 3 µM zoledronic acid (ZA), IL-2 (10³ IU/ml), 100 nM CD80, 0.8 nM IL-15, and 6 nM IL-18 were added, followed by culture in a CO₂ incubator at 37°C for 3 days.

3 days after the start of culture, the aggregated cells were resuspended using a 1000P micropipette and the number of cells was measured. The same amount of cOpTmizer culture medium, IL-2 (10³ IU/ml), 100 nM CD80, and 0.8 nM IL-15 were further added, followed by culture in a CO₂ incubator at 37°C.

4 days after the start of culture, the aggregated cells were resuspended using a 1000P micropipette and the number of cells was measured. 5 days after the start of culture, the aggregated cells were resuspended using a 1000P micropipette and the number of cells was measured. The same amount of cOpTmizer culture medium based on the cell culture fluid in culture, IL-2 (10³ IU/ml), 100 nM CD80, and 0.8 nM IL-15 were further added, followed by culture in a CO₂ incubator at 37°C.

6 days after the start of culture, the aggregated cells were resuspended using a 1000P micropipette and the number of cells was measured. Half of the cOpTmizer culture medium based on the cell culture fluid in culture, IL-2 (10³ IU/ml), 100 nM CD80 and 0.8 nM IL-15 were further added, followed by culture in a CO₂ incubator at 37°C.

7 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells/ml based on the cells in culture, after which IL-2 (10³ IU/ml), 100 nM CD80, and 0.8 nM IL-15 were further added to the additional cOpTmizer culture medium, followed by culture in a CO₂ incubator at 37°C for 3 days.

10 days after the start of culture, the aggregated cells were resuspended using a 1000P micropipette or a 10 ml serological pipette and the number of cells was measured. The cells were diluted with the same amount of cOpTmizer culture medium based on the cell culture fluid in culture, after which IL-2 (10³ IU/ml), 100 nM CD80, and 0.8 nM IL-15 were further added to the additional cOpTmizer culture medium, followed by culture in a CO₂ incubator at 37°C for 2 days.

The same amount of cOpTmizer culture medium based on the cell culture fluid in culture was added and IL-2 (10³ IU/ml), 100 nM CD80, and 0.8 nM IL-15 were further added to the additional cOpTmizer culture medium, followed by culture in a CO₂ incubator at 37°C.

14 days after the start of culture, the aggregated cells were resuspended using a 1000P micropipette and the number of cells was measured. In Example 3, the compositions and proportions of cells including αβ T cells and γδ T cells were analyzed using Fortessa-X20 flow cytometry from BD Bioscience on the start date of culture, day 3, day 4, day 5, day 6, day 7, day 10, and day 14 using the antibodies in Table 1.

Thereby, as shown in FIGs. 5 and 6, for male donors, the proliferation rate of γδ T cells in the experimental group in which finasteride (10 nM), IL-15, IL-18, and CD80 were further added increased about 1.45 times compared to the control group treated with only IL-2 and ZA. On day 14 of culture, the cells were confirmed to be composed of 95% or more of γδ T cells and about 2-3% of αβ T cells.

In addition, as shown in FIGs. 7 and 8, for female donors, the proliferation rate of γδ T cells in the experimental group in which finasteride (0.01 nM), IL-15, IL-18, and CD80 were further added increased about 3.8 times (380%) compared to the control group treated with only IL-2 and ZA.

### Example 4. Proliferation rate and viability of γδ T cells in healthy peripheral blood depending on finasteride treatment period

Peripheral blood mononuclear cells (PBMC) were isolated from healthy donors and were cultured for a total of 14 days by continuous treatment with 10 nM finasteride as the active ingredient for each period until day 7, day 10, and day 14 from the start date of culture, in addition to the basic culture components for γδ T cells (3 µM zoledronic acid and 10³ IU/ml IL-2), and the activation and proliferation rates of γδ T cells were compared.

The frozen peripheral blood mononuclear cells were thawed in a constant temperature water bath at 37°C and then suspended in 9 ml of OpTmizer medium (cOpTmizer, Thermo-Fisher Scientific, USA) containing 10% fetal bovine serum (FBS). The number of cells was measured, followed by centrifugation at 400 × g and 25°C for 4 minutes. The cell pellets obtained by centrifugation were suspended in 5 ml of cOpTmizer medium (Thermo-Fisher Scientific, USA) and the number of cells was measured, after which the cells were diluted with cOpTmizer medium to a concentration of 1.0×10⁶ cells/ml.

A total of 1.0×10⁶ cells was dispensed at 1 ml per well in a 24-well plate. To stimulate and induce differentiation of γδ T cells, 10 nM finasteride, 3 µM zoledronic acid (ZA), and 100 ul of IL-2 (10³ IU/ml) were added, followed by culture in a CO₂ incubator at 37°C for 4 days.

4 days after the start of culture, the aggregated cells were resuspended using a 1000P micropipette, and the same amount of cOpTmizer culture medium based on the cell culture fluid in culture and 10 nM finasteride and IL-2 (10³ IU/ml) for re-induction were further added, followed by culture in a CO₂ incubator at 37°C.

5 days after the start of culture, the aggregated cells were resuspended using a 1000P micropipette and the number of cells was measured. 10 nM finasteride and IL-2 (10³ IU/ml) were further added to the same amount of cOpTmizer culture medium based on the cell culture fluid in culture, followed by culture in a CO₂ incubator at 37°C.

The next day, 10 nM finasteride and IL-2 (10³ IU/ml) were further added to half of the cOpTmizer culture medium based on the cell culture fluid in culture, followed by culture in a CO₂ incubator at 37°C.

7 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells/ml based on the cells in culture, after which 10 nM finasteride and IL-2 (10³ IU/ml) were added to the additional cOpTmizer culture medium, followed by culture in a CO₂ incubator at 37°C for 3 days.

10 days after the start of culture, the aggregated cells were resuspended using a 1000P micropipette or a 10 ml serological pipette and the number of cells was measured. The cells were diluted with cOpTmizer culture medium to a concentration of 0.4×10⁶ cells/ml based on the cells in culture, after which 10 nM finasteride and IL-2 (10³ IU/ml) were added to the additional cOpTmizer culture medium, followed by culture in a CO₂ incubator at 37°C.

On day 12 after the start of the culture, the same amount of cOpTmizer culture medium based on the cell culture fluid in culture, 10 nM finasteride, and IL-2 (10³ IU/ml) were added, followed by culture in a CO₂ incubator at 37°C.

14 days after the start of culture, the aggregated cells were resuspended using a 1000P micropipette or a 10 ml serological pipette and the number of cells was measured.

Peripheral blood mononuclear cells from healthy donors were isolated and were continuously treated with the basic culture components for γδ T cells plus the high concentration of finasteride until day 7, day 10, and day 14, and the activation and proliferation rates of γδ T cells for 14 days were compared.

Thereby, as shown in FIG. 9, the proliferation rate of γδ T cells in the group treated continuously with finasteride until day 7 increased about 1.5 times compared to the control group, and increased at least 1.1 times compared to the group treated continuously until day 10 or 14. There was no significant difference in viability compared to the control group, and continuous treatment with the high concentration of finasteride did not affect cell viability. The proportion of γδ T cells was determined to be about 70% when cultured for 7 days, and 90% or more when cultured for 10 days.

Using the medium containing IL-2 and ZA as a control group, the proliferation rates of γδ T cells by the ingredients added in examples are summarized in Table 2 below.

As shown in Table 2, when finasteride was added, the proliferation rate of γδ T cells increased compared to the control group, and when IL-15 and cytokine mix (IL-15, IL-18, and CD80) were added, the proliferation rate of γδ T cells was confirmed to further increase.

**[Table 2]**

| Comparison of γδ T cell proliferation rates by ingredient added to medium | | |
|---|---|---|
| Ingredient added to medium in addition to IL-2 and ZA | From male donor (10 nM Finasteride) | From female donor (0.01 nM Finasteride) |
| Finasteride (Example 1) | 1.35 times increase | 1.15 times increase |
| Finasteride, IL-15 (Example 2) | 1.26 times increase | 1.11 times increase |
| Finasteride, IL-15, IL-18, CD80 (Example 3) | 1.45 times increase | 3.80 times increase |

### Industrial Applicability

According to the present invention, γδT cells with high cell killing ability and cell viability can be produced in a short period of time with high purity and high efficiency using a clinically friendly method compared to conventionally known general γδ T cell culture methods or culture methods using feeder cells, thus increasing the productivity of allogeneic γδ T cell immunotherapeutic agents.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

## Claims

1. A method of producing γδ T cells, comprising:
(a) inducing and culturing γδ T cells by culturing peripheral blood mononuclear cells (PBMCs) in medium comprising (i) zoledronic acid, (ii) IL-2, and (iii) a 5α-reductase inhibitor or an androgen receptor antagonist; and
(b) obtaining the cultured γδ T cells.

2. The method according to claim 1, wherein the 5α-reductase inhibitor is selected from the group consisting of finasteride, dutasteride, epristeride, alfatradiol, and Saw Palmetto extract.

3. The method according to claim 1, wherein the androgen receptor antagonist is selected from the group consisting of bicalutamide, flutamide, and nilutamide.

4. The method according to claim 1, wherein the 5α-reductase inhibitor is finasteride.

5. The method according to claim 1, wherein the medium further comprises a costimulatory molecule.

6. The method according to claim 5, wherein the costimulatory molecule is an antibody or a ligand to 5A6.E9, Bl, TS8.2, 15D, B6, B3, TS-1, γ3.20, 7A5, IMMU510, R9.12, 11F2 or combinations thereof, phorbol 12-myristate 13-acetate (TPA), mezerein, Staphylococcus enterotoxin A (SEA), Streptococcus protein A, amphotericin B or combinations thereof, αTCR, βTCR, γTCR, δTCR, CD277, CD28, CD46, CTLA4, ICOS, PD-1, CD30, NKG2D, NKG2A, HVEM, 4-1BB (CD137), OX40 (CD134), CD70, CD80, CD86, DAP, CD122, GITR, FceRIg, CD1, CD16, CD161, DNAX, accessory molecule-1 (DNAM-1), SLAM, Coxsackievirus and adenovirus receptors or combinations thereof.

7. The method according to claim 5, wherein the costimulatory molecule is selected from the group consisting of CD80, 4-1BB, CD86, and CD276.

8. The method according to claim 1, wherein the medium further comprises a cytokine selected from the group consisting of IL-4, IL-7, IL-12, IL-15, IL-18, IL-21, and IL-23.

9. The method according to claim 8, wherein the medium further comprises a cytokine selected from the group consisting of IL-12, IL-15, and IL-18.

10. The method according to claim 4, wherein, when the peripheral blood mononuclear cells are derived from males, the medium comprises 1 to 50 nM finasteride.

11. The method according to claim 4, wherein, when the peripheral blood mononuclear cells are derived from females, the medium comprises 0.001 to 10 nM finasteride.

12. The method according to claim 1, wherein the culturing is performed for 3 to 21 days.

13. The method according to claim 1, wherein a proportion of γδ T cells in total cells on day 14 of culture is 90% or more.

14. A medium composition for inducing and culturing γδ T cells, comprising (i) zoledronic acid, (ii) IL-2, and (iii) a 5α-reductase inhibitor or an androgen receptor antagonist.

15. The medium composition according to claim 14, wherein the 5α-reductase inhibitor is selected from the group consisting of finasteride, dutasteride, epristeride, alfatradiol, and Saw Palmetto extract.

16. The medium composition according to claim 14, wherein the androgen receptor antagonist is selected from the group consisting of bicalutamide, flutamide, and nilutamide.

17. The medium composition according to claim 14, wherein the 5α-reductase inhibitor is finasteride.

18. The medium composition according to claim 14, wherein the medium further comprises a costimulatory molecule.

19. The medium composition according to claim 18, wherein the costimulatory molecule is an antibody or a ligand to 5A6.E9, Bl, TS8.2, 15D, B6, B3, TS-1, γ3.20, 7A5, IMMU510, R9.12, 11F2 or combinations thereof, phorbol 12-myristate 13-acetate (TPA), mezerein, Staphylococcus enterotoxin A (SEA), Streptococcus protein A, amphotericin B or combinations thereof, αTCR, βTCR, γTCR, δTCR, CD277, CD28, CD46, CTLA4, ICOS, PD-1, CD30, NKG2D, NKG2A, HVEM, 4-1BB (CD137), OX40 (CD134), CD70, CD80, CD86, DAP, CD122, GITR, FceRIg, CD1, CD16, CD161, DNAX, accessory molecule-1 (DNAM-1), SLAM, Coxsackievirus and adenovirus receptors or combinations thereof.

20. The medium composition according to claim 18, wherein the costimulatory molecule is selected from the group consisting of CD80, 4-1BB, CD86, and CD276.

21. The medium composition according to claim 14, wherein the medium further comprises a cytokine selected from the group consisting of IL-4, IL-7, IL-12, IL-15, IL-18, IL-21, and IL-23.

22. The medium composition according to claim 21, wherein the medium further comprises a cytokine selected from the group consisting of IL-12, IL-15, and IL-18.
